# EUROPEAN PATENT APPLICATION

(11) **EP 4 567 428 A2**
(43) Date of publication of application: **11.06.2025**
(21) Application number: 25157385.3
(22) Date of filing: 11.10.2019
(51) Int. Cl.: G01N 33/68

(54) **TREATMENT WITH HIGHLY SILYLATED IGG COMPOSITIONS**

(30) Priority: 11.10.2018 US 201862744536 P; 29.07.2019 US 201962879930 P
(62) Divisional of application: 19872100.3
(71) Applicant: Momenta Pharmaceuticals, Inc., Titusville, NJ 08560 (US)
(72) Inventor: ARROYO, Santiago, Cambridge, MA 02142 (US); DENNEY, William, Cambridge, MA 02142 (US)
(74) Representative: Mathys & Squire

(57) **Abstract**

Compositions comprising a highly sialylated IgG preparation and methods for treating a patient using such preparations are described.

## Description

### CLAIM OF PRIORITY

This application claims the benefit of U.S. Provisional Application Serial No. 62/744,536, filed on October 11, 2018 and U.S. Provisional Application Serial No. 62/879,930, filed on July 29, 2019. The entire contents of the foregoing are incorporated herein by reference.

### BACKGROUND

Intravenous immunoglobulin (IVIg or IVIG; CAS Number: 9007-83-4) is a commercially available therapeutic product (e.g., Bivigam^{®}, Carimune^{®}, Cuvitru^{®}, Flebogamma^{®}, Gammagard^{®}, GamaSTAN^{®}, Gammaked^{®}, Gammaplex^{®}, Gamunex-C^{®}, Hizentra^{®}, Hyqvia^{®}, Octagam^{®} and Privigen^{®}) primarily composed of human immunoglobulin G (IgG). IVIG is prepared from the pooled plasma of thousands of healthy donors, thus ensuring that the diversity in the IgG repertoire exceeds that of an individual donor. Intravenous immunoglobulin is used to treat a wide variety of chronic autoimmune and systemic inflammatory conditions. Autoimmune indications include idiopathic thrombocytopenic purpura (ITP), Kawasaki disease, Guillain-Barré syndrome and other autoimmune neuropathies, myasthenia gravis, dermatomyositis and several rare diseases. The precise mechanism of action of IVIg is not well-understood. Various studies have documented a series of non-mutually exclusive mechanisms modulating components of the innate and adaptive immune system (4, 6). For example, IVIG has been shown to mediate anti-inflammatory responses through its action on dendritic cells, natural killer cells, regulatory T cells, B cells, and the monocyte/macrophage system, and through its suppression or neutralization of soluble factors, such as inflammatory cytokines, chemokines, and pathogenic autoantibodies.

### SUMMARY

This application is based, in part, on the surprising discovery that a dose of a highly sialylated IgG (hsIgG) preparation that is about 1% - 10% of the effective dose for IVIG can be effective for treating disorders that are treated with IVIG. A highly sialylated IgG preparation can be prepared from IVIG Thus, similar IVIG, it includes a heterogeneous mixture of IgG subclasses and a wide array of antibodies expected to be present in human serum. When hsIgG is prepared from IVIG, the large number of donors ensures diversity in the Ig repertoire.

A highly sialylated IgG (hsIgG) preparation is an IgG preparation in which at least 60% of the branched glycans on the IgG antibodies have a sialic acid (i.e., are sialylated) on both the alpha 1,3 and alpha 1,6 branch that is connected through a NeuAc-α 2,6-Gal terminal linkage. Put differently, at least 60% of the branched glycans have a sialic acid on each branch and this sialic acid is NeuAc and is linked to Gal by an α2,6 linkage. IgG antibodies have a glycosylation site at position N297 of the Fc domain and in the highly sialylated IgG preparation at least 60% of the branched glycans on the Fc domain of the IgG antibodies have a sialic acid on both the alpha 1,3 and alpha 1,6 branch that is connected through a NeuAc-α 2,6-Gal terminal linkage. The IgG antibodies can also have branched glycans on the Fab region and at least 50% of these branched glycans have a sialic acid on both the alpha 1,3 and alpha 1,6 branch that is connected through a NeuAc-α 2,6-Gal terminal linkage.

The hsIgG preparation, when prepared from IVIG, can also be referred to as highly as highly sialylated IVIGor hyper sialylated IVG preparation (hsIVIG).

In some embodiments, the hsIgG preparation used in the methods described herein comprises IgG wherein at least 60% (70%, 75%, 80%, 85%, 90%, or 95%) of the branched glycans on the IgG have a sialic acid on both the α1,3 branch and the α1,6 branch. In some embodiments, at least 65%, 70%, 75%, 80%, 85%, 90% or 95% of the Fc glycans on the IgG have a sialic on both the α1,3 branch and the α1,6 branch. In some embodiments, at least 50%, 55%, 60%, 65%, 70%, 75%, 80%, or 85% of the Fab branched glycans on IgG have a sialic acid on both the α1,3 branch and the α1,6 branch. In some cases, at least 80%, of the branched glycans on the IgG have a sialic acid on both the α1,3 branch and the α1,6 branch. In some embodiments, at least 85% of the Fc glycans on the IgG have a sialic on both the α1,3 branch and the α1,6 branch. In some embodiments, at least 60% of the Fab branched glycans on IgG have a sialic acid on both the α1,3 branch and the α1,6 branch.

In some embodiments, at least 90%, 92%, 93%, 94% or 95% w/w of the proteins in the hsIgG preparation are IgG.

In some embodiments, the invention relates to a method for treating a disorder, the method comprising administering a composition comprising a hsIgG preparation to a subject at a dose that is 1% - 10% of the effective dose for IVIG In some embodiments, the effective dose of IVIG is 400 mg/kg, 500 mg/kg, 600 mg/kg, 1000 mg/kg, or 2000 mg/kg. In some embodiments, a composition comprising hsIgG preparation is administered at a dose of about 2, 3, 4, 5, 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200, 250, 300, 350, 400, 450, 500, 550, 600, 650, 700, 750, 800, 850, 900, 950, 975, or 1000 mg/kg. In some embodiments, a composition comprising hsIgG preparation is administered daily, weekly, semiweekly, biweekly, monthly, semimonthly, bimonthly, every 3 days, every 4 days, every 5 days, every 6 days, every 7 days, once every 14 days, once every 21 days, once every 28 days, once daily for two consecutive days in a 28-day cycle, or with the same administration frequency as the FDA approved IVIG dose. In some cases, the hsIgG preparation is administered less often than the effective or approved administration frequency for IVIG. In some embodiments, a composition comprising hsIgG preparation is administered intravenously, subcutaneously, or intramuscularly. In some embodiments, a composition is administered in a single dose. In some embodiments, a composition is administered in multiple doses.

In some embodiments, the disorder is an inflammatory disorder. In some embodiments, the subject is suffering from antibody deficiency. In some embodiments, the subject is suffering from primary antibody deficiency. In some embodiments, the disorder is associated with the presence of autoantibodies.

In some embodiments, the disorder is a neurological disorder. In some embodiments, the neurological disorder is selected from the group consisting of: dermatomyositis, Guillain-Barre syndrome, chronic inflammatory demyelinating polyneuropathy (CIDP), multifocal motor neuropathy (MMN), myasthenia gravis and stiff person syndrome.

In some embodiments, the disorder is selected from the group consisting of: immune cytopenias, parvovirus B19 associated red cell aplasia, hypogammaglobulinaemia secondary to myeloma and chronic lymphatic leukaemia and post-bone marrow transplantation.

In some embodiments, the disorder is selected from the group consisting of: sculitis, systemic lupus erythematosis (SLE), mucous membrane pemphigoid and uveitis and in dermatology it is used most commonly to treat Kawasaki syndrome, dermatomyositis, toxic epidermal necrolysis and the blistering diseases.

In some embodiments, the disorder is FDA-approved for treatment with IVIG. In some embodiments, the dose is 1% - 10% (e.g., 1% - 10%, 1-9%, 1-8%, 1-7%, 1-6%, 1-5%, 1-4%, 1-3%, 1-2%, 2 - 10%, 2-9%, 2-8%, 2-7%, 2-6%, 2-5%, 2-4%, 2-3%, 3-10%, 3-9%, 3-8%, 3-7%, 3-6%, 3-5%, 3-4%, 1-2%, 3%, 2% or 1%) of the FDA approved IVIG dose for the disorder. In some embodiments, the FDA approved dose of IVIG is 200 mg/kg 400 mg/kg, 500 mg/kg, 600 mg/kg, 1000 mg/kg, or 2000 mg/kg. In some embodiments, a composition comprising a hsIgG preparation is administered at a dose of about 2, 3, 4, 5, 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200, 250, 300, 350, 400, 450, 500, 550, 600, 650, 700, 750, 800, 850, 900, 950, 975, or 1000 mg/kg. In some embodiments, a composition comprising a hsIgG preparation is administered daily, weekly, semiweekly, biweekly, monthly, semimonthly, bimonthly, every 3 days, every 4 days, every 5 days, every 6 days, every 7 days, once every 14 days, once every 21 days, once every 28 days, once daily for two consecutive days in a 28-day cycle, or with the same administration frequency as the FDA approved IVIG dose. In some embodiments, a composition comprising hsIgG preparation is administered intravenously, subcutaneously, or intramuscularly. In some embodiments, a composition is administered in a single dose. In some embodiments, a composition is administered in multiple doses.

In some embodiments, the disorder is selected from the group consisting of:
Myocarditis
Acute motor axonal neuropathy
Adiposis dolorosa
Anti-Glomerular Basement Membrane nephritis; Goodpasture syndrome
Antiphospholipid syndrome (APS, APLS)
Antisynthetase syndrome; Myositis, ILD
ataxic neuropathy (acute & chronic)
Autoimmune enteropathy (AIE)
Autoimmune neutropenia
Autoimmune retinopathy
Autoimmune thyroiditis
Autoimmune urticaria
Dermatitis herpetiformis
Epidermolysis bullosa acquisita
Essential mixed cryoglobulinemia
Granulomatosis with polyangiitis(GPA)
Mixed connective tissue disease(MCTD)
Neuromyotonia
Optic neuritis
Paraneoplastic cerebellar degeneration
Anti-N-Methyl-D-Aspartate (Anti-NMDA) Receptor Encephalitis
Autoimmune hemolytic anemia
Autoimmune thrombocytopenic purpura
Chronic inflammatory demyelinating polyneuropathy
Dermatomyositis
Gestational pemphigoid
Graves' disease
Guillain-Barré syndrome
IgG4-related disease
Lambert-Eaton myasthenic syndrome
Lupus nephritis
Myositis
Multifocal motor neuropathy
Myasthenia gravis
Neuromyelitis optica
Pemphigus vulgaris
Polymyositis and
Systemic Lupus Erythematosus (SLE).

In some embodiments, the disorder is selected from the group consisting of:
Acute disseminated encephalomyelitis (ADEM)
Autoimmune Angioedema (Acquired angioedema type II)
Autoimmune hepatitis (Type I & Type II)
Autoimmune hypophysitis; Lymphocytic hypophysitis
Autoimmune inner ear disease (AIED)
Evans syndrome
Graves ophthalmopathy
Hashimoto's encephalopathy
IgA vasculitis (IgAV)
Latent autoimmune hepatitis
Linear IgA disease (LAD)
Lupus vasculitis
Membranous glomerulonephritis
Microscopic polyangiitis (MPA)
Mooren's ulcer
Morphea
Opsoclonus myoclonus syndrome
Ord's thyroiditis
Palindromic rheumatism
Paraneoplastic opsoclonus - myoclonus-ataxia with neuroblastoma
Pediatric Autoimmune Neuropsychiatric Disorder Associated with Streptococcus (PANDAS)
Postpericardiotomy syndrome
Primary biliary cirrhosis (PBC)
Rasmussen Encephalitis
Rheumatoid vasculitis
Schnitzler syndrome
Sydenham chorea
Undifferentiated connective tissue disease (UCTD), and
Miller Fisher Syndrome.

In some embodiments, the composition comprises a hsIgG preparation wherein at least 60% of the branched glycans on the Fab domain have a sialic acid on both the α 1,3 arm and the α 1,6 arm that is connected through aNeuAc-α 2,6-Gal terminal linkage; and at least 60% of the branched glycan on the Fc domain have a sialic acid on both the α 1,3 arm and the α 1,6 arm that is connected through a NeuAc-α 2,6-Gal terminal linkage.

Disclosed herein is a method of treating CIDP in a subject having CIDP comprising by administering a hsIgG preparation at a dose of 10% or less than 10% of the of the effective dose for IVIG In some embodiments, the effective dose for IVIG is 200-2000 mg/kg. In some embodiments, the hsIgG preparation is administered at a dose of 10% of the effective dose for IVIG In some embodiments, the hsIgG preparation is administered at a dose of 1% - 10%, 1-9%, 1-8%, 1-7%, 1-6%, 1-5%, 1-4%, 1-3%, 1-2% or 1% of the effective dose for IVIG In some embodiments, the a hsIgG preparation is administered at a dose of about 2, 3, 4, 5, 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 mg/kg.

Disclosed herein is a method of ITP in a subject having ITP comprising administering a hsIgG preparation at a dose of 10% or less than 10% of the effective dose for IVIG In some embodiments, the effective dose for IVIG is 1000-2000 mg/kg mg/kg. In some embodiments, the a hsIgG preparation is administered at a dose of 10% of the effective dose for IVIG In some embodiments, the a hsIgG preparation is administered at a dose of 1% - 10%, 1-9%, 1-8%, 1-7%, 1-6%, 1-5%, 1-4%, 1-3%, 1-2%, 2 - 10%, 2-9%, 2-8%, 2-7%, 2-6%, 2-5%, 2-4%, 2-3%, 3-10%, 3-9%, 3-8%, 3-7%, 3-6%, 3-5%, 3-4%, 1-2%, 2% or 1% of the effective dose for IVIG In some embodiments, the a hsIgG preparation is administered at a dose of about 2, 3, 4, 5, 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 mg/kg.

Disclosed herein is a method of wAIHA in a subject having wAIHA comprising administering a hsIgG preparation at a dose of 10% or less than 10%of the effective dose for IVIG In some embodiments, the effective dose for IVIG is 1000 mg/kg. In some embodiments, the hsIgG preparation is administered at a dose of 1% - 10%, 1-9%, 1-8%, 1-7%, 1-6%, 1-5%, 1-4%, 1-3%, 1-2%, 2 - 10%, 2-9%, 2-8%, 2-7%, 2-6%, 2-5%, 2-4%, 2-3%, 3-10%, 3-9%, 3-8%, 3-7%, 3-6%, 3-5%, 3-4%, 1-2%, 2% or 1% of the effective dose for IVIG In some embodiments, the hsIgG preparation is administered at a dose of 1% of the effective dose for IVIG In some embodiments, the hsIgG preparation is administered at a dose of about 2, 3, 4, 5, 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 mg/kg.

Disclosed herein is a method of Guillain-Barre Syndrome in a subject having Guillain-Barre Syndrome comprising administering hsIgG preparation at a dose of 10% of the of the effective dose for IVIG. In some embodiments, the effective dose for IVIG is 1000-2000 mg/kg. In some embodiments, the hsIgG preparation is administered at a dose of less than 10% of the effective dose for IVIG In some embodiments, the hsIgG preparation is administered at a dose of 1% - 10%, 1-9%, 1-8%, 1-7%, 1-6%, 1-5%, 1-4%, 1-3%, 1-2%, 2 - 10%, 2-9%, 2-8%, 2-7%, 2-6%, 2-5%, 2-4%, 2-3%, 3-10%, 3-9%, 3-8%, 3-7%, 3-6%, 3-5%, 3-4%, 1-2%, 2% or 1% of the effective dose for IVIG. In some embodiments, the a hsIgG preparation is administered at a dose of about 2, 3, 4, 5, 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 mg/kg.

Disclosed herein is a method of PID (primary humoral immunodeficiency disease) in a subject having PID comprising administering a hsIgG preparation at a dose of 10% or less than 10% of the effective dose for IVIG. In some embodiments, the effective dose for IVIG is 200-800 mg/kg. In some embodiments, the hsIgG preparation is administered at a dose of 1% - 10%, 1-9%, 1-8%, 1-7%, 1-6%, 1-5%, 1-4%, 1-3%, 1-2%, 2 - 10%, 2-9%, 2-8%, 2-7%, 2-6%, 2-5%, 2-4%, 2-3%, 3-10%, 3-9%, 3-8%, 3-7%, 3-6%, 3-5%, 3-4%, 1-2%, 2% or 1% of the effective dose for IVIG. In some embodiments, the hsIgG preparation is administered at a dose of 1% of the effective dose for IVIG. In some embodiments, the a hsIgG preparation is administered at a dose of about 2, 3, 4, 5, 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 mg/kg.

Disclosed herein is a method of method of treating Kawasaki disease in a subject having Kawasaki disease comprising administering a hsIgG preparation at a dose of 10% or less than 10% of the of the effective dose for IVIG In some embodiments, the effective dose for IVIG is 1000-2000 mg/kg. In some embodiments, the hsIgG preparation is administered at a dose of 1% - 10%, 1-9%, 1-8%, 1-7%, 1-6%, 1-5%, 1-4%, 1-3%, 1-2%, 2 - 10%, 2-9%, 2-8%, 2-7%, 2-6%, 2-5%, 2-4%, 2-3%, 3-10%, 3-9%, 3-8%, 3-7%, 3-6%, 3-5%, 3-4%, 1-2%, 2% or 1% of the effective dose for IVIG In some embodiments, the hsIgG preparation is administered at a dose of 1% of the effective dose for IVIG In some embodiments, the a hsIgG preparation is administered at a dose of about 2, 3, 4, 5, 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 mg/kg.

In some embodiments, administering a composition comprising a hsIgG preparation at a dose that is 1% - 10%, 1-9%, 1-8%, 1-7%, 1-6%, 1-5%, 1-4%, 1-3%, 1-2%, 2 - 10%, 2-9%, 2-8%, 2-7%, 2-6%, 2-5%, 2-4%, 2-3%, 3-10%, 3-9%, 3-8%, 3-7%, 3-6%, 3-5%, 3-4%, 1-2%, 2% or 1% of the effective dose for IVIG has similar efficacy to administering a composition comprising the effective dose of IVIG.

In some embodiments, at least one side effect attributed to the effective dose for IVIG is alleviated by administering a hsIgG preparation at a dose that is 1% - 10% of the effective dose for IVIG In some embodiments, administration of a composition comprising a hsIgG preparation, as compared to the effective dose of IVIQ results in the reduction in severity or time of one or more of the following side effects: swelling, pain, discoloration of a limb, shortness of breath, rapid pulse/tachycardia, numbness or weakness in a limb or one side of the body, brown or red urine, yellowing of eyes or skin, fever over 100 °F, dizziness, muscle cramps, nausea, vomiting, myalgia, and hypotension.

As used herein, "glycan" is a sugar, which can be monomers or polymers of sugar residues, such as at least three sugars, and can be linear or branched. A "glycan" can include natural sugar residues (e.g., glucose, N-acetylglucosamine, N-acetyl neuraminic acid, galactose, mannose, fucose, hexose, arabinose, ribose, xylose, etc.) and/or modified sugars (e.g., 2'-fluororibose, 2'-deoxyribose, phosphomannose, 6'sulfo N-acetylglucosamine, etc.). The term "glycan" includes homo and heteropolymers of sugar residues. The term "glycan" also encompasses a glycan component of a glycoconjugate (e.g., of a glycoprotein, glycolipid, proteoglycan, etc.). The term also encompasses free glycans, including glycans that have been cleaved or otherwise released from a glycoconjugate.

As used herein, the term "Fc region" refers to a dimer of two "Fc polypeptides", each "Fc polypeptide" comprising the constant region of an antibody excluding the first constant region immunoglobulin domain. In some embodiments, an "Fc region" includes two Fc polypeptides linked by one or more disulfide bonds, chemical linkers, or peptide linkers. "Fc polypeptide" refers to the last two constant region immunoglobulin domains of IgA, IgD, and IgG, and the last three constant region immunoglobulin domains of IgE and IgM, and may also include part or all of the flexible hinge N-terminal to these domains. For IgG, "Fc polypeptide" comprises immunoglobulin domains Cgamma2 (Cy2) and Cgamma3 (Cγ3) and the lower part of the hinge between Cgamma1 (Cγ1) and Cy2. Although the boundaries of the Fc polypeptide may vary, the human IgG heavy chain Fc polypeptide is usually defined to comprise residues starting at T223 or C226 or P230, to its carboxyl-terminus, wherein the numbering is according to the EU index as in Kabat et al. (1991, NIH Publication 91-3242, National Technical Information Services, Springfield, VA). For IgA, Fc polypeptide comprises immunoglobulin domains Calpha2 (Cα2) and Calpha3 (Cα3) and the lower part of the hinge between Calpha1 (Cα1) and Cα2. An Fc region can be synthetic, recombinant, or generated from natural sources such as IVIG

As used herein, an "N-glycosylation site of an Fc region" refers to an amino acid residue within an Fc region to which a glycan is N-linked.

For any given parameter, in some embodiments, "percent" refers to the number of moles of a particular glycan (glycan X) relative to total moles of glycans of a preparation. The percent can, in some cases, be assessed by determining the number of moles of PNGase F-released Fc glycan X relative to total moles of PNGase F-released Fc glycans.

By "purified" (or "isolated") refers to a nucleic acid sequence (e.g., a polynucleotide) or an amino acid sequence (e.g., a polypeptide) that is removed or separated from other components present in its natural environment. For example, an isolated polypeptide is one that is separated from other components of a cell in which it was produced (e.g., the endoplasmic reticulum or cytoplasmic proteins and RNA). An isolated polynucleotide is one that is separated from other nuclear components (e.g., histones) and/or from upstream or downstream nucleic acid sequences. An isolated nucleic acid sequence or amino acid sequence can be at least 60% free, or at least 75% free, or at least 90% free, or at least 95% free from other components present in natural environment of the indicated nucleic acid sequence or amino acid sequence.

As used herein, the term "ST6 sialyltransferase" refers to a polypeptide whose amino acid sequence includes at least one characteristic sequence of and/or shows at least 100%, 99%, 98%, 97%, 96%, 95%, 94%, 93%, 92%, 91%, 90% identity with a protein involved in transfer of a sialic acid to a terminal galactose of a glycan through an α2,6 linkage (e.g., ST6 Gal-I). A wide variety of ST6 sialyltransferase sequences are known in the art, such as those described herein; in some embodiments, an ST6 sialyltransferase shares at least one characteristic sequence of and/or shows the specified degree of overall sequence identity with one of the ST6 sialyltransferases set forth herein (each of which may be considered a "reference" ST6 sialyltransferase). In some embodiments, an ST6 sialyltransferase as described herein shares at least one biological activity with a reference ST6 sialyltransferase as set forth herein. In some such embodiment, the shared biological activity relates to transfer of a sialic acid to a glycan. A suitable ST6 sialyltransferase for preparing hsIgG is human ST6Gal, which can be expressed in CHO cells.

### N-Linked Glycosylation

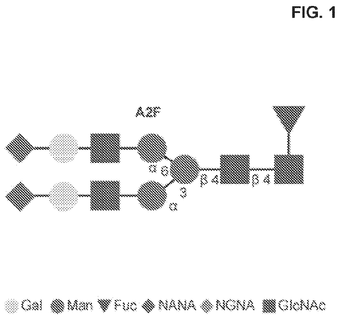

N-linked oligosaccharide chains are added to a protein in the lumen of the endoplasmic reticulum (see Molecular Biology of the Cell, Garland Publishing, Inc. (Alberts et al., 1994)). Specifically, an initial oligosaccharide (typically 14-sugar) is added to the amino group on the side chain of an asparagine residue contained within the target consensus sequence of Asn-X-Ser/Thr, where X may be any amino acid except proline. The structure of this initial oligosaccharide is common to most eukaryotes, and contains 3 glucose, 9 mannose, and 2 N-acetylglucosamine residues. This initial oligosaccharide chain can be trimmed by specific glycosidase enzymes in the endoplasmic reticulum, resulting in a short, branched core oligosaccharide composed of two N-acetylglucosamine and three mannose residues (depicted in Figure 1, linked to an asparagine residue). One of the branches is referred to in the art as the "α1,3 arm", and the second branch is referred to as the "α1,6 arm", as denoted in Figure 1.

N-glycans can be subdivided into three distinct groups called "high mannose type", "hybrid type", and "complex type", with a common pentasaccharide core (Man (alpha1,6)-(Man(alpha1,3))-Man(beta1,4)-GlcpNAc(beta 1,4)-GlcpNAc(beta 1,N)-Asn) occurring in all three groups.

After initial processing in the endoplasmic reticulum, the glycoprotein is transported to the Golgi where further processing may take place. If the glycan is transferred to the Golgi before it is completely trimmed to the core pentasaccharide structure, it results in a "high-mannose glycan".

Additionally or alternatively, one or more monosaccharides units of N-acetylglucosamine may be added to core mannose subunits to form a "complex glycan". Galactose may be added to N-acetylglucosamine subunits, and sialic acid subunits may be added to galactose subunits, resulting in chains that terminate with any of a sialic acid, a galactose or an N-acetylglucosamine residue. Additionally, a fucose residue may be added to an N-acetylglucosamine residue of the core oligosaccharide. Each of these additions is catalyzed by specific glycosyl transferases, known in the art.

Sialic acids are a family of 9-carbon monosaccharides with heterocyclic ring structures. They bear a negative charge via a carboxylic acid group attached to the ring as well as other chemical decorations including N-acetyl and N-glycolyl groups. The two main types of sialyl residues found in glycoproteins produced in mammalian expression systems are N-acetyl-neuraminic acid (NeuAc) and N-glycolylneuraminic acid (NeuGc). These usually occur as terminal structures attached to galactose (Gal) residues at the nonreducing termini of both N- and O-linked glycans. The glycosidic linkage configurations for these sialyl groups can be either α2,3 or α2,6.

"Hybrid glycans" comprise characteristics of both high-mannose and complex glycans. For example, one branch of a hybrid glycan may comprise primarily or exclusively mannose residues, while another branch may comprise N-acetylglucosamine, sialic acid, and/or galactose sugars.

When referring to a certain percent of the effective dose of IVIG, the specified percent refers to a range of ± 5 mg/kg. Thus, 10% of a 1,000 mg/kg dose is 1000 mg/kg ± 5% and 5% of a 1,000 mg/kg dose is 50 mg/kg ±5%.

Antibodies are glycosylated at conserved, N-linked glycosylation sites in the Fc regions of immunoglobulin heavy chains. For example, each heavy chain of an IgG antibody has a single N-linked glycosylation site at Asn297 of the CH2 domain (see Jefferis, Nature Reviews 8:226-234 (2009)). IgA antibodies have N-linked glycosylation sites within the CH2 and CH3 domains, IgE antibodies have N-linked glycosylation sites within the CH3 domain, and IgM antibodies have N-linked glycosylation sites within the CH1, CH2, CH3, and CH4 domains (see Arnold et al., J. Biol. Chem. 280:29080-29087 (2005); Mattu et al., J. Biol. Chem. 273:2260-2272 (1998); Nettleton et al., Int. Arch. Allergy Immunol. 107:328-329 (1995)).

Each antibody isotype has a distinct variety of N-linked carbohydrate structures in the constant regions. For example, IgG has a single N-linked biantennary carbohydrate at Asn297 of the CH2 domain in each Fc polypeptide of the Fc region, which also contains the binding sites for C1q and FcyR (see Jefferis et al., Immunol. Rev. 163:59-76 (1998); and Wright et al., Trends Biotech 15:26-32 (1997)). For human IgG, the core oligosaccharide normally consists of GlcNAc2Man3GlcNAc, with differing numbers of outer residues. Variation among individual IgG can occur via attachment of galactose and/or galactose-sialic acid at one or both terminal GlcNAc or via attachment of a third GlcNAc arm (bisecting GlcNAc), and/or attachment of fucose.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

### DESCRIPTION OF DRAWINGS

FIG 1 is a schematic depiction of an example of a branched glycan that is sialylated on both the α 1,3 arm and the α 1,6 arm by way of NeuAc-α 2,6-Gal terminal linkage.

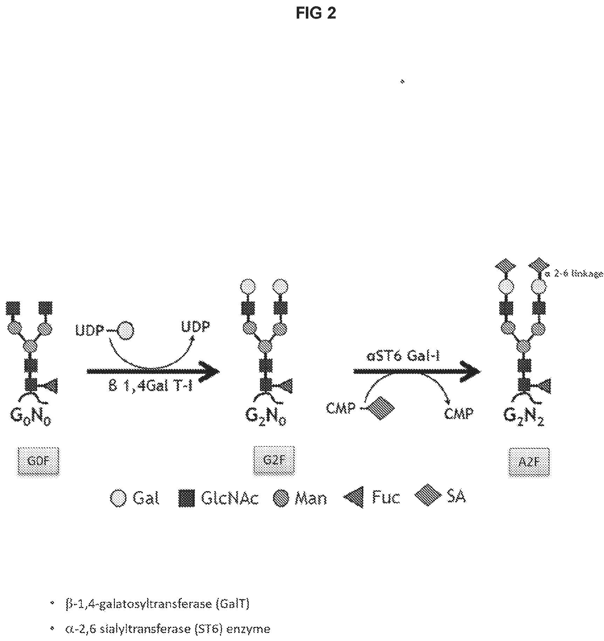

FIG 2 is a schematic illustration of a method for preparing hsIgG The beginning substrate can be IVIG or a fraction thereof.
FIG 3 is a schematic illustration of the sialylation of a branched glycan by ST6Ga11.
FIG 4 is a graph showing the time course of IVIg Fc glycoform proportions in the presence of CMP-NANA and ST6Gal1. Galactosylated IVIg was incubated with 20 mM CMP-NANA and 0.3 U/mg ST6Ga11 at 37 °C. Aliquots were removed at different time points, and the relative proportions of IVIg glycoforms were determined by glycopeptide LC-MS/MS analyses.
FIG 5A and B depicts the results of an analysis of IVIG dose-response in a murine ITP model (ns, Not significant. *P < 0.05; **P < 0.01; ***P < 0.001)
FIG 6 depicts the results of a study comparing of therapeutic dosing of 0.1 g/kg hsIgG with IVIg at 0.1 and 1 g/kg in a murine model of ITP.
FIG 7 depicts the results of a comparison of hsIgG and IVIG in a human patient with ITP.

### DETAILED DESCRIPTION

The present disclosure concerns methods of treatment using a hsIgG preparation. A hsIgG preparation is a preparation comprising a mixture of IgG antibodies (e.g., prepared from IVIG) in which at least 60% of the branched glycans are disialylated, i.e., have a sialic acid on the α1,3 arm (e.g., with a NeuAc-α2,6-Gal terminal linkage) and on the α1,6 arm (e.g., with a NeuAc-α2,6-Gal terminal linkage).

An hsIgG preparation disclosed herein can be used to treat disorders that are treated with IVIG. Importantly, the hsIgG preparations are far more potent than IVIG, permitting effective treatment at a far lower dose and/or less frequent treatment. For example, as shown herein An hsIgG preparation can be 10 to 100-times more potent than IVIG allowing treatment at a dose that is 1% - 10% of the effective or approved IVIG dose.

Current treatments utilizing IVIG have distinct limitations, which include, but are not limited to, variable efficacy, clinical risks, high costs, and finite supply. At the current maximal dosing regimens, only partial and poorly sustained responses are obtained in many instances. In addition, the long infusion times (4-6 h) associated with the high volume of IVIg treatment (IVIG is commonly dosed at 1,000 - 2,000 mg/kg) consume significant resources at infusion centers and negatively affect patient-reported outcomes, such as convenience and quality of life.

A hsIgG preparation, because it is so much more potent than IVIG, can provide shorter infusion times, a far lower amount of protein administered, and, in some cases, less frequent administration. For this reason, a hsIgG preparation can provide a greatly improved patient experience and higher quality of life compared to treatment with IVIG. In some cases, conditions which must be treated with intravenous administration of IVIG can be treated with subcutaneous administration (injection or infusion) of hsIgG, e.g., by means of a subcutaneous pump, allowing administration by the patient at home. In addition, an effective dose of hsIgG can be prepared from a far lower amount of IVIG, than would be required for an effective dose of IVIG This is an important advantage because IVIG is costly in limited supply.

The level of sialylation can be measured on an individual Fc region (e.g., the number of branched glycans that have a sialic acid an α1,3 arm, an α1,6 arm, or both, of the branched glycans in the Fc region), or on the overall composition of a preparation of glycoproteins (e.g., the number or percentage of branched glycans that are have a sialic acid on an α1,3 arm, an α1,6 arm, or both, of the branched glycans in the Fc region in a preparation of glycoproteins).

### Methods of Sialylating IgG preparation

As described in Washburn et al. (Proc Natl Acad Sci 112(11):E1297-306), ST6 GalI sialyltransferase catalyzes the transfer of sialic acid from a sialic acid donor (e.g., cytidine 5'-monophospho-N-acetyl neuraminic acid) to a terminal galactose residue of glycans through an α2,6 linkage in an ordered fashion. ST6 sialyltransferase transfers a sialic acid to an α1,3 arm of a branched glycan, which can be followed by transfer of a second sialic acid to an α1,6 arm (yielding a disialylated branched glycan), and can further be followed by removal of sialic acid from an α1,3 arm (yielding a branched glycan having a sialic acid on an α1,6 arm). Accordingly, by controlling and/or modulating activity (e.g., kinetics) of ST6 sialyltransferase, glycoproteins having particular sialylation patterns can be produced.

Methods for producing hsIgG are described, for example in Washburn et al. and in US 2016/0108450, hereby incorporated by reference. Deletion mutants of ST6Gal-1 sialyltransferase (Engel et al., BMC Proceedings 7(Suppl 6):P110, 2013) can also be useful for preparing hsIgG

A different sialylation method, which fails to meaningfully increase Fab sialylation, is described in Huang et al. (J Am Chem Soc 134(29):12308-12318, 2012). Despite the lack of Fab sialylation, IVIG sialylated by this method may be useful.

A hsIgG preparation can be manufactured from commercially available IVIG as follows. Bulk IVIG, 10% solution (0.1 g/mL), is pooled and diluted with 3-(N-morpholino) propanesulfonic acid (MOPS) pH 7.4 buffer. The solution is diafiltered (DF) with five diavolumes (DVs) of MOPS pH 7.4 buffer using 30 kDa tangential flow filtration (TFF) membranes (membranes composed of polyethersulfone). The buffer exchanged IVIG solution is then concentrated, followed by depth and 0.2 µm filtrations resulting in a final IVIG concentration of ≥ 150 mg/mL. Sialylation is accomplished in two enzymatic reaction steps using two sugar nucleotides dissolved in MOPS pH 7.4 buffer. First, galactosylation occurs by reaction with beta-1,4 galactosyltransferase enzyme (B4GalT), uridine diphosphate galactose (UDP-Gal) and manganese chloride in MOPS pH 7.4 buffer. The reaction solution is adjusted to approximately 135 mg/mL by addition of MOPS buffer and is maintained at approximately 37°C for approximately 48 hours. Following galactosylation, the material is further incubated for approximately 72 hours with the addition of human α 2,6-sialyltransferase enzyme (ST6-Gal1) and cytidine 5'monophospho-N-acetyl neuraminic acid (CMP-NANA), and adjusted to approximately 120 mg/mL with MOPS buffer pH 7.4. CMP-NANA is charged portion wise over the course of reaction at approximately 12 hour intervals.

### Glycan Evaluation

Glycans of glycoproteins can be evaluated using any methods known in the art. For example, sialylation of glycan compositions (e.g., level of branched glycans that are have a sialic acid on an α1,3 arm and/or an α1,6 arm) can be characterized using methods described in, e.g., Barb, Biochemistry 48:9705-9707 (2009); Anumula, J. Immunol. Methods 382:167-176 (2012); Gilar et al., Analytical Biochem. 417:80-88 (2011); Wuhrer et al., J. Chromatogr. B. 849:115-128 (2007). In some embodiments, in addition to evaluation of sialylation of glycans, one or more parameters described in Table 1 are evaluated.

In some instances, glycan structure and composition as described herein are analyzed, for example, by one or more, enzymatic, chromatographic, mass spectrometry (MS), chromatographic followed by MS, electrophoretic methods, electrophoretic methods followed by MS, nuclear magnetic resonance (NMR) methods, and combinations thereof. Exemplary enzymatic methods include contacting a glycoprotein preparation with one or more enzymes under conditions and for a time sufficient to release one or more glycan(s) (e.g., one or more exposed glycan(s)). In some instances, the one or more enzymes include(s) PNGase F. Exemplary chromatographic methods include, but are not limited to, Strong Anion Exchange chromatography using Pulsed Amperometric Detection (SAX-PAD), liquid chromatography (LC), high performance liquid chromatography (HPLC), ultra performance liquid chromatography (UPLC), thin layer chromatography (TLC), amide column chromatography, and combinations thereof. Exemplary mass spectrometry (MS) include, but are not limited to, tandem MS, LC-MS, LC-MS/MS, matrix assisted laser desorption ionisation mass spectrometry (MALDI-MS), Fourier transform mass spectrometry (FTMS), ion mobility separation with mass spectrometry (IMS-MS), electron transfer dissociation (ETD-MS), and combinations thereof. Exemplary electrophoretic methods include, but are not limited to, capillary electrophoresis (CE), CE-MS, gel electrophoresis, agarose gel electrophoresis, acrylamide gel electrophoresis, SDS-polyacrylamide gel electrophoresis (SDS-PAGE) followed by Western blotting using antibodies that recognize specific glycan structures, and combinations thereof. Exemplary nuclear magnetic resonance (NMR) include, but are not limited to, one-dimensional NMR (1D-NMR), two-dimensional NMR (2D-NMR), correlation spectroscopy magnetic-angle spinning NMR (COSY-NMR), total correlated spectroscopy NMR (TOCSY-NMR), heteronuclear single-quantum coherence NMR (HSQC-NMR), heteronuclear multiple quantum coherence (HMQC-NMR), rotational nuclear overhauser effect spectroscopy NMR (ROESY-NMR), nuclear overhauser effect spectroscopy (NOESY-NMR), and combinations thereof.

In some instances, techniques described herein may be combined with one or more other technologies for the detection, analysis, and or isolation of glycans or glycoproteins. For example, in certain instances, glycans are analyzed in accordance with the present disclosure using one or more available methods (to give but a few examples, see Anumula, Anal. Biochem., 350(1):1, 2006; Klein et al., Anal. Biochem., 179:162, 1989; and/or Townsend, R.R. Carbohydrate Analysis" High Performance Liquid Chromatography and Capillary Electrophoresis., Ed. Z. El Rassi, pp 181-209, 1995; WO2008/128216; WO2008/128220; WO2008/128218; WO2008/130926; WO2008/128225; WO2008/130924; WO2008/128221; WO2008/128228; WO2008/128227; WO2008/128230; WO2008/128219; WO2008/128222; WO2010/071817; WO2010/071824; WO2010/085251; WO2011/069056; and WO2011/127322, each of which is incorporated herein by reference in its entirety). For example, in some instances, glycans are characterized using one or more of chromatographic methods, electrophoretic methods, nuclear magnetic resonance methods, and combinations thereof. In some instances, methods for evaluating one or more target protein specific parameters, e.g., in a glycoprotein preparation, e.g., one or more of the parameters disclosed herein, can be performed by one or more of following methods.

In some instances, methods for evaluating one or more target protein specific parameters, e.g., in a glycoprotein preparation, e.g., one or more of the parameters disclosed herein, can be performed by one or more of following methods.

**Table 1: Exemplary methods of evaluating parameters:**

| **Method(s)** | **Relevant Literature** | **Parameter** |
|---|---|---|
| C18 UPLC Mass Spec.* | Chen and Flynn, Anal. Biochem., 370:147-161 (2007) | Glycan(s) (e.g., N-linked glycan, exposed N-linked glycan, glycan detection, glycan identification, and characterization; site specific glycation; glycoform detection (e.g., parameters 1-7); percent glycosylation; and/or aglycosyl) |
| | Chen and Flynn, J. Am. Soc. Mass Spectrom., 20:1821-1833 (2009) | |
| Peptide LC-MS (reducing/non-reducing) | Dick et al., Biotechnol. Bioeng., 100:1132-1143 (2008) | C-terminal lysine |
| | Yan et al., J. Chrom. A., 1164:153-161 (2007) | |
| | Chelius et al., Anal. Chem., 78:2370-2376 (2006) | |
| | Miller et al., J. Pharm. Sci., 100:2543-2550 (2011) | |
| LC-MS (reducing/nonreducing/alkylated) | Dick et al., Biotechnol. Bioeng., 100:1132-1143 (2008) | C-terminal lysine |
| | Goetze et al., Glycobiol., 21:949-959 (2011) | |
| Weak cation exchange (WCX) chromatography | Dick et al., Biotechnol. Bioeng., 100:1132-1143 (2008) | C-terminal lysine |
| LC-MS (reducing/non-reducing/alkylated) | Dick et al., Biotechnol. Bioeng., 100:1132-1143 (2008) | N-terminal pyroglu |
| | Goetze et al., Glycobiol., 21:949-959 (2011) | |
| PeptideLC-MS (reducing/non-reducing) | Yan et al., J. Chrom. A., 1164:153-161 (2007) | N-terminal pyroglu |
| | Chelius et al., Anal. Chem., 78:2370-2376 (2006) | |
| | Miller et al., J. Pharm. Sci., 100:2543-2550 (2011) | |
| Peptide LC-MS (reducing/non-reducing) | Yan et al., J. Chrom. A., 1164:153-161 (2007); | Methionine oxidation |
| | Xie et al., mAbs, 2:379-394 (2010) | |
| Peptide LC-MS (reducing/non-reducing) | Miller et al., J. Pharm. Sci., 100:2543-2550 (2011) | Site specific glycation |
| Peptide LC-MS (reducing/non-reducing) | Wang et al., Anal. Chem., 83:3133-3140 (2011); | Free cysteine |
| | Chumsae et al., Anal. Chem., 81:6449-6457 (2009) | |
| Bioanalyzer (reducing/non-reducing)* | Forrer et al., Anal. Biochem., 334:81-88 (2004) | Glycan (e.g., N-linked glycan, exposed N-linked glycan) (including, for example, glycan detection, identification, and characterization; site specific glycation; glycoform detection; percent glycosylation; and/or aglycosyl) |
| LC-MS (reducing/non-reducing/alkylated)* | Dick et al., Biotechnol. Bioeng., 100:1132-1143 (2008) | Glycan (e.g., N-linked glycan, exposed N-linked glycan) (including, for example, glycan detection, identification, and characterization; site specific glycation; glycoform detection; percent glycosylation; and/or aglycosyl) |
| * Methods include removal (e.g., enzymatic, chemical, and physical) of glycans | Goetze et al., Glycobiol., 21:949-959 (2011) | |
| | Xie et al., mAbs, 2:379-394 (2010) | |
| Bioanalyzer (reducing/non-reducing) | Forrer et al., Anal. Biochem., 334:81-88 (2004) | Light chain : Heavy chain |
| Peptide LC-MS (reducing/non-reducing) | Yan et al., J. Chrom. A., 1164:153-161 (2007) | Non-glycosylation-related peptide modifications (including, for example, sequence analysis and identification of sequence variants; oxidation; succinimide; aspartic acid; and/or site-specific aspartic acid) |
| | Chelius et al., Anal. Chem., 78:2370-2376 (2006) | |
| | Miller et al., J. Pharm. Sci., 100:2543-2550 (2011) | |
| Weak cation exchange (WCX) chromatography | Dick et al., Biotechnol. Bioeng., 100:1132-1143 | Isoforms (including, for example, charge variants |
| | (2008) | (acidic variants and basic variants); and/or deamidated variants) |
| Anion-exchange chromatography | Ahn et al., J. Chrom. B, 878:403-408 (2010) | Sialylated glycan |
| Anion-exchange chromatography | Ahn et al., J. Chrom. B, 878:403-408 (2010) | Sulfated glycan |
| 1,2-diamino-4,5-methylenedioxybenzene (DMB) labeling method | Hokke et al., FEBS Lett., 275:9-14 (1990) | Sialic acid |
| LC-MS | Johnson et al., Anal. Biochem., 360:75-83 (2007) | C-terminal amidation |
| LC-MS | Johnson et al., Anal. Biochem., 360:75-83 (2007) | N-terminal fragmentation |
| Circular dichroism spectroscopy | Harn et al., Current Trends in Monoclonal Antibody Development and Manufacturing, S. J. Shire et al., eds, 229-246 (2010) | Secondary structure (including, for example, alpha helix content and/or beta sheet content) |
| Intrinsic and/or ANS dye fluorescence | Harn et al., Current Trends in Monoclonal Antibody Development and Manufacturing, S. J. Shire et al., eds, 229-246 (2010) | Tertiary structure (including, for example, extent of protein folding) |
| Hydrogen-deuterium exchange-MS | Houde et al., Anal. Chem., 81:2644-2651 (2009) | Tertiary structure and dynamics (including, for example, accessibility f amide protons to solvent water) |
| Size-exclusion chromatography | Carpenter et al., J. Pharm. Sci., 99:2200-2208 (2010) | Extent of aggregation |
| Analytical ultracentrifugation | Pekar and Sukumar, Anal. Biochem., 367:225-237 (2007) | |

The literature recited above are hereby incorporated by reference in their entirety or, in the alternative, to the extent that they pertain to one or more of the methods for determining a parameter described herein.

### Pharmaceutical Compositions and Administration

A hsIgG can be incorporated into a pharmaceutical composition. Pharmaceutical compositions for intravenous administration of a hsIgG preparation can be formulated by methods known to those skilled in the art. For example, the pharmaceutical composition can be formulated by suitably combining the hsIgG preparation with pharmaceutically acceptable vehicles or media, such as sterile water and physiological saline, vegetable oil, emulsifier, suspension agent, surfactant, stabilizer, flavoring excipient, diluent, vehicle, preservative, binder, followed by mixing in a unit dose form required for generally accepted pharmaceutical practices. The amount of active ingredient included in the pharmaceutical preparations is such that a suitable dose within the designated range is provided.

The sterile composition for injection can be formulated in accordance with conventional pharmaceutical practices using distilled water for injection as a vehicle. For example, physiological saline or an isotonic solution containing glucose and other supplements such as D-sorbitol, D-mannose, D-mannitol, and sodium chloride may be used as an aqueous solution for injection, optionally in combination with a suitable solubilizing agent, for example, alcohol such as ethanol and polyalcohol such as propylene glycol or polyethylene glycol, and a nonionic surfactant such as polysorbate 80^{™}, HCO-50 and the like.

Non-limiting examples of oily liquid include sesame oil and soybean oil, and it may be combined with benzyl benzoate or benzyl alcohol as a solubilizing agent. Other items that may be included are a buffer such as a phosphate buffer, or sodium acetate buffer, a soothing agent such as procaine hydrochloride, a stabilizer such as benzyl alcohol or phenol, and an antioxidant. The formulated injection can be packaged in a suitable ampule.

In some embodiments, the dose is 1% - 10% of the FDA approved (or other national or international regulatory agency) IVIG dose or the effective IVIG dose for a disorder. In some embodiments, the FDA (or other national or international regulatory agency) approved dose or effective dose of IVIG is 200 mg/kg, 400 mg/kg, 500 mg/kg, 600 mg/kg, 1000 mg/kg, or 2000 mg/kg. In some embodiments, a composition comprising a hsIgG preparation is administered at a dose of about 4, 5, 6, 10, 15, 20, 30, 40, 50, 60, 70, 80, 90, 100, 110, 120, 130, 140, 150, 160, 170, 180, 190, 200, 250, 300, 350, 400, 450, 500, 550, 600, 650, 700, 750, 800, 850, 900, 950, 975, or 1000 mg/kg. In some embodiments, a composition comprising a hsIgG preparation is administered daily, weekly, semiweekly, biweekly, monthly, semimonthly, bimonthly, every 3 days, every 4 days, every 5 days, every 6 days, every 7 days, once every 14 days, once every 21 days, once every 28 days, once daily for two consecutive days in a 28-day cycle, or with the same administration frequency as the FDA approved IVIG dose. In some embodiments, a composition is administered in a single dose. In some embodiments, a composition is administered in multiple doses. The dose and method of administration varies depending on the weight, age, condition, and the like of the patient, and can be suitably selected as needed by those skilled in the art.

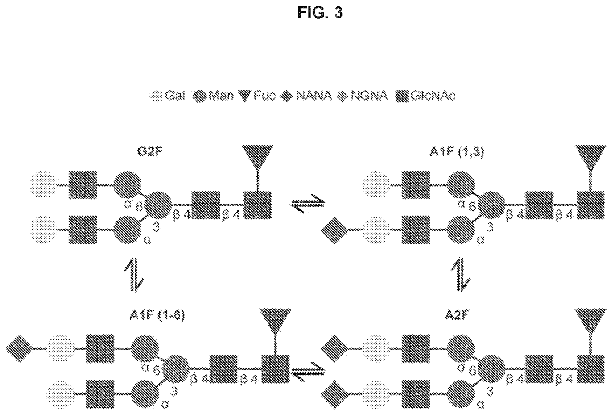

As described in Washburn et al., analysis of sialylation by ST6Ga11 revealed that ST6Ga11 can not only catalyze the transfer of the sialic acid from CMP-NANA sugar nucleotide to the Fc glycan but also, facilitate the removal of the sialic acid from the sialylated product. The reactions are shown schematically in FIG. 3.

As Washburn et al. reported, under certain reaction conditions, sialylation of the α1,3 branch of the biantennary glycan (to form A1F-1,3) was rapid and essentially complete by 30 min, whereas the doubly sialylated species (A2F) formed at an ~10× slower rate and stopped accumulating by 24 h. After 24 h, a monosialylated species with the sialic acid on the α1,6 branch(A1F-1,6) started to form and continued accumulating steadily, reaching ~35% of glycosylated species by 64 h. Concomitantly, A2F exhibited a steady decline from 71% at 20 h to 44% at 64 h, suggesting that the A1F-1,6 glycoform was generated from the removal of sialic acid residues on the more exposed α1,3 branch of disialylated A2F. Additional incubation led to the cleavage of the 1,6 sialic acid in the A1F-1,6 glycoform, generating the asialylated but fully galactosylated and fucosylated species G2F. G2F, which was present in trace amounts at the beginning of the reaction, appeared at measurable amounts at 40 h and continued increasing, reaching 15% by 64 h as the levels of A2F and A1F-1,6 declined.

Washburn et al. further reported that these observations were critical in optimizing the parameters to maximize the yield of the A2F species while minimizing the A1F-1,3, A1F-1,6, and G2F glycoforms. By evaluating a matrix of parameters affecting the transient state of the G2F 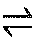 A1F-1,3 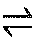 A2F 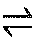 A1F-1,6 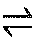 G2F glycoform distribution, they found that the desialylation component of the reaction (A2F 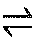 A1F-1,6 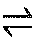 G2F) was facilitated by spontaneous decomposition of CMP-NANA in the reaction. Wasburn et al. concluded that replenishing the CMP-NANA in the sialylation reaction assists in maximize the A2F yield and found that periodic dosing of fresh CMP-NANA maximized the A2F glycan levels after 24 h without the formation of substantial A1F-1,6 or G2F species.

### Galactosylation and sialylation of IVIG

The sialylation of IVIG by the sialyltransferase ST6 was analyzed. IVIG was first galactosylated and then sialylated. The reactions were performed sequentially. There was no purification between galactosylation and sialylation reactions. The relative abundance of glycoforms was analyzed following the sialylation reactions.

### A. Galactosylation

A reaction was set up that contained the following components at the concentrations indicated:

| **Constituent** | **Final concentration** |
|---|---|
| MOPS (pH 7.4) | 25 mM |
| MnCl₂ | 10 mM |
| IVIG | 12.5 mg/ml |
| B4GalT1 (90 u/ml) | 400 mu/ml |
| UDP-Galactose | 50 mM |

The reaction was incubated for 72 hours at 37 °C.

### B. Sialylation

To an aliquot of the galactosylation reaction were added CMP-NANA, MOPS buffer and ST6Gal1. The final volume was adjusted so that the final concentration of components in the reaction was as indicated.

| **Constituent** | **Final concentration** |
|---|---|
| MOPS (pH 7.4) | 50 mM |
| MnCl₂ | 8 mM |
| IVIG | 10 mg/ml |
| CMP-NANA | 20 mM |
| ST6Ga11 (SEQ ID NO:1) | 0.6 mg ST6/mg |

The reaction was incubated at 37 °C. Aliquots were extracted at various times frozen at -20 °C for later analyses.

### C. Results

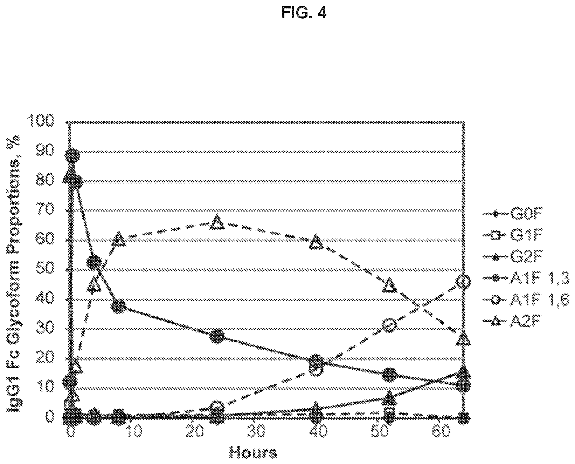

FIG. 4 depicts the time course of IVIG Fc domain glycoform proportions over the course of a sialylation reaction performed essentially as described above in the presence of CMP-NANA and ST6Gal1. Briefly, galactosylated IVIG was incubated with 20 mM CMP-NANA and 0.3 U/mg ST6Ga11 at 37 °C. Aliquots were removed at different time points, and the relative proportions of IVIG glycoforms were determined by glycopeptide LC-MS/MS analyses. As shown in FIG. 4, the predominant glycoform changed over time from G2F to A1F (1,3) to A2F to A1F (1,6) during the course of a reaction due to competing addition (forward reaction) and removal (back reaction) steps.

### Analysis of a hsIgG preparation

A hsIgG preparation manufactured from commercially available IVIG was prepared essentially as described above with periodic addition of CMP-NANA during the sialylation reaction. The starting IVIG material and the resulting hsIgG preparation were extensively analyzed before and after sialylation. With regard to Fc domain sialylation, the hsIgG preparation was substantially tetrasialylated (i.e., the branched glycan on each Fc chain was sialylated on both branches. More detailed analyses of glycosylation were performed in an isotype- and site-specific manner to discriminate the different Fc isotypes and Fab glycosylation. Fc glycosylation was changed from predominantly asialylated species (e.g., G0F and G1F) in the starting IVIG material to more than 90% disialylated species (e.g., A2F, A2, and A2F+BGlcNAc) in each IgG isotype in the hsIgG preparation. Analysis of the Fab glycans further revealed that, although the IVIG starting material contained a significant distribution of monosialylated (~35%) and disialylated (~35%) glycans in the Fab, the distribution shifted to a higher level of disialylated glycans in the hsIgG preparation (~75%).

### hsIgG is about 10 times potent than IVIG in prevent platelet destruction in a murine model of ITP

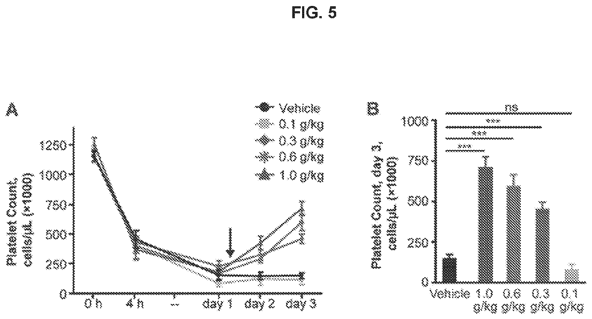

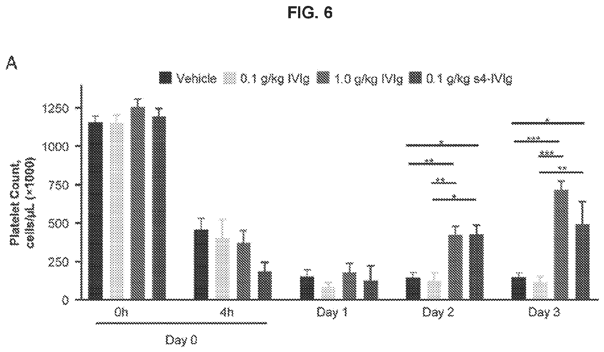

Washburn et al. compared a hsIgG preparation to IVIG in a murine anti-CD41 antibody-induced thrombocytopeania model. Briefly, 6A6-IgG2a antibodies were produced by transient transfection of 293T cells followed by purification of recombinant antibodies from serum-free cell culture supernatants with protein G beads (GE Healthcare) as suggested by the manufacturer. IVIG preparations were diluted in glycine buffer (saline) for experiments. Chronic ITP was induced by daily injections of 0.1 µg/g 6A6-IgG2a antiplatelet antibody. Mice were rendered thrombocytopenic until the end of the experiment (day 3). Platelet counts were determined before and 4 h after daily antibody injection of a 1:4 dilution in PBS in a hematology system (Advia 120; Bayer HealthCare). Platelet counts before antibody injection were set to 100%. A dose-response profile for IVIG in the ITP model (FIG. 5). After maximum platelet depletion with the antiplatelet antibody, mice were treated with IVIG from 0.1 to 1 g/kg, and platelet levels were measured on the day of the IVIg treatment (day 1) and the two subsequent days after treatment (days 2 and 3 of the experiment). A clear dose-response was observed in this model within this range (FIG. 5). Complete activity of IVIg was lost at 0.1 g/kg. Comparing the hsIgG preparation and IVIG in the ITP model, a clear enhancedefficacy was observed. As shown in FIG. 6, therapeutic treatment with hsIgG at 0.1 g/kg returned platelet levels at days 2 and 3 to levels similar to those obtained with IVIg at 1 g/kg.

### Studies in human patients

A hsIgG preparation (at least 80% of the branched glycans were sialylated on both the α 1,3 arm and the α 1,6 arm by way of NeuAc-α 2,6-Gal terminal linkages) was compared to IVIG in human patients with ITP. In one study, a single ascending dose was given to normal healthy volunteers over a 3.5 month period. In this double-blind, placebo controlled study, patients were given a single dose of hsIgG every 14 days in the following order: 3 mg/kg, 10 mg/kg, 30 mg/kg, 60 mg/kg, 120 mg/kg, and 250 mg/kg. End point measurements were safety and tolerability.

In a separate study, a single ascending dose was given to ITP patients over a 4 month period. ITP patients were given a single dose of hsIVIG in the following fixed order: 60 mg/kg, 120 mg/kg, 250 mg/kg, 500 mg/kg, and 1000 mg/kg. The hsIgG dose was administered, and after 28 days, 1000 mg/kg IVIG was administered to the same patient. Platelet levels were measured throughout the study. End point measurements were safety and tolerability.

In a randomized study, ITP patients were started on either hsIgG or IVIG and switched mid-study. In one subsection, 5 patients were started on a high hsIVIG dose and another 5 patients were started on 1,000 mg/kg IVIG. Midway through to 2 month study, the patients that started on a high hsIVIG dose were given 1,000 mg/kg IVIG for the remainder of the study, and the patients that started on 1,000 mg/kg IVIG were given a high hsIgG dose for the remainder of the study. Platelet response was measured throughout. In anothe subsection, 5 patients were started on a low hsIgG dose and another 5 patients were started on 1,000 mg/kg IVIG. Midway through to 2 month study, the patients that started on a low hsIgG dose were given 1,000 mg/kg IVIG for the remainder of the study, and the patients that started on 1,000 mg/kg IVIG were given a low hsIG dose for the remainder of the study. Platelet response was measured throughout. The potency of the hsIVIG was determined.

### A hsIgG preparation is more potent than IVIG in human ITP patient

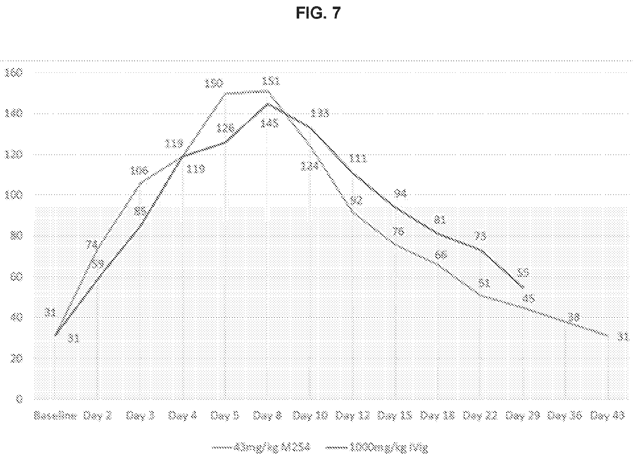

A hsIgG preparation was compared to IVIG in a human patient with ITP. In this hsIgG preparation at least 80% of the branched glycans were sialylated on both the α 1,3 arm and the α 1,6 arm by way of NeuAc-α 2,6-Gal terminal linkages. The patient was dosed with 43mg/kg hsIVIG. As shown in FIG 7, this patient had similar reticulocyte count when administered a hsIgG preparation at 43 mg/kg as at the 1000 mg/kg IVIG dose. Thus, the hsIgG preparation was about 25-fold more potent than IVIG in preventing platelet destruction in this patient.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.
Also disclosed are the following aspects:
1. A method for treating a disorder, the method comprising administering a hsIgG preparation to a subject at a dose that is 1% - 10% of the effective dose of IVIG for treating the disorder.
2. The method of aspect 1, wherein the hsIgG preparation is administered at a dose of 5 mg/kg to 100 mg/kg.
3. The method of aspect 1, wherein the disorder is an inflammatory disorder.
4. The method of aspect 1, wherein the subject is suffering from antibody deficiency.
5. The method of aspect 4, wherein the subject is suffering from primary antibody deficiency.
6. The method of aspect 1, wherein the disorder is associa aspect th the presence of autoantibodies.
7. The method of aspect 1 wherein the dose of hsIVIG is as effective as the effective dose of IVIG.
8. The method of aspect 1 or aspect 7, wherein the hsIgG preparation is administered at the same frequency as the effective dose of IVIG.
9. The method of aspect 1, wherein the disorder is a neurological disorder.
10. The method of aspect 9, wherein the neurological disorder is selected from the group consisting of: dermatomyositis, Guillain-Barre syndrome, chronic inflammatory demyelinating polyneuropathy (CIDP), multifocal motor neuropathy (MMN), myasthenia gravis and stiff person syndrome.
11. The method of aspect 1, wherein the disorder is selected from the group consisting of: immune cytopenias, parvovirus B19 associated red cell aplasia, hypogammaglobulinaemia secondary to myeloma and chronic lymphatic leukaemia and post-bone marrow transplantation.
12. The method of aspect 1, wherein the disorder is selected from the group consisting of: sculitis, systemic lupus erythematosis (SLE), mucous membrane pemphigoid and uveitis and in dermatology it is used most commonly to treat Kawasaki syndrome, dermatomyositis, toxic epidermal necrolysis and the blistering diseases.
13. The method of aspect 1, wherein the disorder is FDA-approved for treatment with IVIG or IVIG is indicated for treatment of the disorder.
14. The method of aspect 13, wherein the hsIgG preparation preparation is 1% - 10% of the FDA approved IVIG dose for the disorder.
15. The method of aspect 1, wherein the disorder is selected from the group consisting of:
   Myocarditis
   Acute motor axonal neuropathy
   Adiposis dolorosa
   Anti-Glomerular Basement Membrane nephritis; Goodpasture syndrome
   Antiphospholipid syndrome (APS, APLS)
   Antisynthetase syndrome; Myositis, ILD
   ataxic neuropathy (acute & chronic)
   Autoimmune enteropathy (AIE)
   Autoimmune neutropenia
   Autoimmune retinopathy
   Autoimmune thyroiditis
   Autoimmune urticaria
   Dermatitis herpetiformis
   Epidermolysis bullosa acquisita
   Essential mixed cryoglobulinemia
   Granulomatosis with polyangiitis(GPA)
   Mixed connective tissue disease(MCTD)
   Neuromyotonia
   Optic neuritis
   Paraneoplastic cerebellar degeneration
   Anti-N-Methyl-D-Aspartate (Anti-NMDA) Receptor Encephalitis
   Autoimmune hemolytic anemia
   Autoimmune thrombocytopenic purpura
   Chronic inflammatory demyelinating polyneuropathy
   Dermatomyositis
   Gestational pemphigoid
   Graves' disease
   Guillain-Barré syndrome
   IgG4-related disease
   Lambert-Eaton myasthenic syndrome
   Lupus nephritis
   Myositis
   Multifocal motor neuropathy
   Myasthenia gravis
   Neuromyelitis optica
   Pemphigus vulgaris
   Polymyositis and
   Systemic Lupus Erythematosus (SLE).
16. The method of aspect 1, wherein the disorder is selected from the group consisting of:
   Acute disseminated encephalomyelitis (ADEM)
   Autoimmune Angioedema (Acquired angioedema type II)
   Autoimmune hepatitis (Type I & Type II)
   Autoimmune hypophysitis; Lymphocytic hypophysitis
   Autoimmune inner ear disease (AIED)
   Evans syndrome
   Graves ophthalmopathy
   Hashimoto's encephalopathy
   IgA vasculitis (IgAV)
   Latent autoimmune hepatitis
   Linear IgA disease (LAD)
   Lupus vasculitis
   Membranous glomerulonephritis
   Microscopic polyangiitis (MPA)
   Mooren's ulcer
   Morphea
   Opsoclonus myoclonus syndrome
   Ord's thyroiditis
   Palindromic rheumatism
   Paraneoplastic opsoclonus - myoclonus-ataxia with neuroblastoma
   Pediatric Autoimmune Neuropsychiatric Disorder Associated with
   Streptococcus (PANDAS)
   Postpericardiotomy syndrome
   Primary biliary cirrhosis (PBC)
   Rasmussen Encephalitis
   Rheumatoid vasculitis
   Schnitzler syndrome
   Sydenham chorea
   Undifferentiated connective tissue disease (UCTD), and
   Miller Fisher Syndrome.
17. The method of any of the forgoing aspects, wherein 60% of the glycans on the IgG in the hsIgG preparation have a sialic acid on both the α1,3 branch and the α1,6 branch.
18. The method of aspect 17, wherein at least 60% of the branched glycans on the Fab domain have a sialic acid on both the α 1,3 arm and the α 1,6 arm that is connected through a NeuAc-α 2,6-Gal terminal linkage; and at least 60% of the branched glycan on the Fc domain have a sialic acid on both the α 1,3 arm and the α 1,6 arm that is connected through a NeuAc-α 2,6-Gal terminal linkage.
19. A method of treating CIDP in a subject having CIDP comprising administering a hsIgG preparation at an effective dose than is 10% or less than 10% of the effective dose for IVIG.
20. The method of aspect 19, wherein the effective dose for IVIG is 200-2000 mg/kg.
21. The method of aspect 19 or 20, wherein the hsIgG preparation is administered at an effective dose that is 10% or less than 10% of the effective dose for IVIG.
22. The method of aspect 19 or 20, wherein the hsIgG preparation is administered at a dose of 1% of the effective dose for IVIG.
23. The method of aspect 19, wherein the hsIgG preparation is administered at a dose of about 2, 3, 4, 5, 6, 7, 8, 9, 10, 15, 20, 25, 30, 35, 40, 45, 50, 55, 60, 65, 70, 75, 80, 90, 100, 110, 120, 130, 140, 150, 160, 170, 180, 190, or 200 mg/kg.
24. A method of treating ITP in a subject having ITP comprising administering a hsIgG preparation at an effective dose that is 10% or less than 10% of the of the effective dose for IVIG.
25. The method of aspect 24, wherein the effective dose for IVIG is 1000-2000 mg/kg mg/kg.
26. The method of aspect 24, wherein the hsIgG preparation is administered at an effective dose that is 10% or less than 10% of the effective dose for IVIG.
27. The method of aspect 24, wherein the hsIgG preparation is administered at a dose of 1%-5% of the effective dose for IVIG.
28. The method of aspect 24, wherein the hsIgG preparation is administered at a dose of about 10, 20, 30, 40, 50, 60, 70, 80, 90, 100, 110, 120, 130, 140, 150, 160, 170, 180, 190, or 200 mg/kg.
29. A method of treating wAIHA in a subject having wAIHA comprising administering a hsIgG preparation at an effective dose that is 10% or less than 10% of the of the effective dose for IVIG.
30. The method of aspect 29, wherein the effective dose for IVIG is 1000 mg/kg.
31. The method of aspect 29, wherein the hsIgG preparation is administered at a dose of less than 10% of the effective dose for IVIG.
32. The method of aspect 29, wherein the hsIgG preparation is administered at a dose of 1%-5% of the effective dose for IVIG.
33. The method of aspect 29, wherein the hsIgG preparation is administered at a dose of about 10, 20, 30, 40, 50, 60, 70, 80, 90, or 100 mg/kg.
34. A method of treating Guillain-Barre Syndrome in a subject having Guillain-Barre Syndrome comprising administering a hsIgG preparation at an effective dose that i 10% or less than 10% of the of the effective dose for IVIG.
35. The method of aspect 34, wherein the effective dose for IVIG is 1000-2000 mg/kg.
36. The method of aspect 34, wherein the hsIgG preparation is administered at a dose of 10% of the effective dose for IVIG.
37. The method of aspect 34, wherein the hsIgG preparation is administered at a dose of 1%-5% of the effective dose for IVIG.
38. The method of aspect 34, wherein the hsIgG preparation is administered at a dose of about 10, 20, 30, 40, 50, 60, 70, 80, 90, 100, 110, 120, 130, 140, 150, 160, 170, 180, 190, or 200 mg/kg.
39. A method of treating PID (primary humoral immunodeficiency disease) in a subject having PID comprising administering the hsIgG preparation at an effective dose that is 10% or less than 10% of the of the effective dose for IVIG.
40. The method of aspect 39, wherein the effective dose for IVIG is 200-800 mg/kg.
41. The method of aspect 39, wherein the hsIgG preparation is administered at a dose of less than 10% of the effective dose for IVIG.
42. The method of aspect 39, wherein the hsIgG preparation is administered at a dose of 1%-5% of the effective dose for IVIG.
43. The method of aspect 39, wherein the hsIgG preparation is administered at a dose of about 2, 3, 4, 5, 6, 7, 8, 9, 10, 15, 20, 25, 30, 35, 40, 45, 50, 55, 60, 65, 70, 75, or 80 mg/kg.
44. A method of treating Kawasaki disease in a subject having Kawasaki disease comprising administering a hsIgG preparation at an effective dose that is10% or less than 10% of the of the effective dose for IVIG.
45. The method of aspect 44, wherein the effective dose for IVIG is 1000-2000 mg/kg.
46. The method of aspect 44, wherein the hsIgG preparation is administered at a dose of less than 10% of the effective dose for IVIG.
47. The method of aspect 44, wherein the hsIgG preparation is administered at a dose of 1%-5% of the effective dose for IVIG.
48. The method of aspect 44, wherein the hsIgG preparation is administered at a dose of about 10, 20, 30, 40, 50, 60, 70, 80, 90, 100, 110, 120, 130, 140, 150, 160, 170, 180, 190, or 200 mg/kg.
49. The method of any one of the preceding aspects, wherein the dose of a hsIgG preparation has similar efficacy to the effective dose for IVIG.
50. The method of any one of the preceding aspects, wherein at least one side effect attributed to the effective dose for IVIG is alleviated by administering the a hsIgG preparation.
51. An article of manufacture comprising a container comprising a sterile composition comprising 110, 9, 8, 7, 6, 5, 4, 3, 2 or 1 mg of a hsIgG preparation.
52. The article of manufacture of aspect 51, wherein at least 60% of the branched glycans on the Fab domain of the IgG in the hsIgG preparation are have a sialic acid on both the α 1,3 arm and the α1,6 arm that is connected through a NeuAc-α 2,6-Gal terminal linkage; and at least 60% of the branched glycan on the Fc domain of the IgG in the hsIgG preparation have a sialic acid on both the α 1,3 arm and the α 1,6 arm by way of NeuAc-α 2,6-Gal terminal linkage.
53. The article of manufacture of aspect 51, wherein the container is a vial, bottle or bag.

## Claims

1. An hsIgG preparation for use in a method of treating chronic inflammatory demyelinating polyneuropathy (CIDP) in a subject having CIDP, the method comprising administering the hsIgG preparation at an effective dose of 10% or less than 10% of the dose of IVIG effective for treating CIDP.

2. The hsIgG preparation for use according to claim 1, wherein the method comprises administering the hsIgG preparation to the subject at a dose that is 1% - 10% of the effective dose of IVIG for treating CIDP.

3. The hsIgG preparation for use according to claim 2, wherein the hsIgG preparation is administered at a dose of 5 mg/kg to 100 mg/kg.

4. The hsIgG preparation for use according to claim 2, wherein the dose of hsIgG is as effective in treating CIDP as the dose of IVIG effective in treating CIDP.

5. The hsIgG preparation for use according to claim 2 or claim 4, wherein the hsIgG preparation is administered at the same frequency as the dose of IVIG effective in treating CIDP.

6. The hsIgG preparation for use according to claim 2, wherein the hsIgG preparation is 1% - 10% of the FDA approved IVIG dose for CIDP.

7. The hsIgG preparation for use according to any one of claims 2-6, wherein 60% of the glycans on the IgG in the hsIgG preparation have a sialic acid on both the α1,3 branch and the α1,6 branch.

8. The hsIgG preparation for use according to claim 7, wherein at least 60% of the branched glycans on the Fab domain of the IgG have a sialic acid on both the α1,3 arm and the α1,6 arm connected through a NeuAc-α2,6-Gal terminal linkage; and at least 60% of the branched glycan on the Fc domain of the IgG have a sialic acid on both the α1,3 arm and the α1,6 arm connected through a NeuAc-α2,6-Gal terminal linkage.

9. The hsIgG preparation for use according to any one of the preceding claims, wherein the effective dose of IVIG for treating CIDP is 200-2000 mg/kg.

10. The hsIgG preparation for use according to any one of the preceding claims, wherein the hsIgG preparation is administered at a dose of 1% of the dose of IVIG effective for treating CIDP.

11. The hsIgG preparation for use according to claim 1, wherein the hsIgG preparation is administered at a dose of 2, 3, 4, 5, 6, 7, 8, 9, 10, 15, 20, 25, 30, 35, 40, 45, 50, 55, 60, 65, 70, 75, 80, 90, 100, 110, 120, 130, 140, 150, 160, 170, 180, 190, or 200 mg/kg.

12. The hsIgG preparation for use according to any one of the preceding claims, wherein the dose of the hsIgG preparation has similar efficacy to the dose of IVIG effective for treating CIDP.

13. The hsIgG preparation for use according to any one of the preceding claims, wherein at least one side effect attributed to the effective dose for IVIG is alleviated by administering the hsIgG preparation at a dose that is 1%-10% of the effective dose for IVIG for treating CIDP.
